# EUROPEAN PATENT APPLICATION

(11) **EP 1 361 433 A2**
(43) Date of publication of application: **12.11.2003**
(21) Application number: 03252225.2
(22) Date of filing: 08.04.2003
(51) Int. Cl.: G01N 33/48

(54) **Method for estimating therapeutic efficacy of tumor necrosis factor (TNF)**

(30) Priority: 09.04.2002 JP 2002107126
(71) Applicant: KABUSHIKI KAISHA HAYASHIBARA SEIBUTSU KAGAKU KENKYUJO, Okayama-shi Okayama (JP)
(72) Inventor: Yanai, Yoshiaki, c/ KK Hayashibara Seibutsu Kagaku, Okayama-shi, Okayama 700-0907 (JP); Yamamoto, Shigeto, c/o KK Hayashibara Seibutsu, Okayama-shi, Okayama 700-0907 (JP); Yamamoto, Kozo, c/o KK Hayashibara Seibutsu, Okayama-shi, Okayama 700-0907 (JP); Ikegami, Hakuo, c/o KK Hayashibara Seibutsu, Okayama-shi, Okayama 700-0907 (JP)
(74) Representative: Daniels, Jeffrey Nicholas

(57) **Abstract**

Disclosed is a method for estimating the therapeutic efficacy of physiologically active substances or antitumor agents, particularly, tumor necrosis factor (TNF-α) in the treatment of cancers. The method comprises a step of evaluating the expression level of a TNF-α-related gene in a cancer cell.

## Description

### Background of the Invention

### Field of the Invention

The present invention relates to a method for estimating the potential therapeutic efficacy of physiologically active substances or anti-tumor agents, particularly, tumor necrosis factor (hereinafter abbreviated as "TNF-α" throughout the specification) in the treatment of cancers.

### Description of the Prior Art

Treatments with antitumor agents have been used in medical fields as effective therapeutic methods for treating cancers. Conventional antitumor agents, however, are only effective on restricted types of cancers, and this narrows their applicability. In addition, there are not so many antitumor agents with satisfactory therapeutic efficacy. Most of conventional antitumor agents have relatively strong side effects, and this results in a heavy burden to cancer patients as a serious demerit.

Recently, as novel antitumor agents, the use of physiologically active substances have been highlighted. Comparing with conventional antitumor agents mainly produced by chemical synthesis, such physiologically active substances have the merit that they have a relatively wide applicability because of their satisfactory antitumor effects on more various types of cancers. Among the physiologically active substances, particularly, TNF-α, which was discovered by L. J. Old et al. in 1975 as a cytotoxic factor secreted in sera of animals such as rabbits and mice which had been sequentially administered with BCG and intracellular toxin, has been focused on as a physiologically active substance with a strong antitumor activity on a variety of antitumor cells since the discovery. However, even the above TNF-α has serious side effects similarly as in conventional antitumor agents and subsidiary acts on some types of cells to cause fever, and therefore, TNF-α has not yet been actually used in medical fields.

Due to the genetic progress such as the total human gene analysis as a result of the worldwide project, gene-related screening tools such as DNA microarray technology have been rapidly progressed. *Jikken-Igaku*, extra number, edited by H. Aburatani, Vol. 19, No. 19, pp. 2,518-2,524 (2001) discloses a trial of applying the analysis of gene expression profiles using DNA microarray technology to accurately evaluate the properties of cancers in diagnosing cancers; and *Jikken-Igaku*, extra number, edited by K. Chiba et al, Vol. 19, No. 19, pp. 2,507-2,512 (2001) proposes a trial of estimating the individual difference in effects of pharmaceuticals such as antitumor agents, i.e., pharmacogenomics. Since the above trials would directly enable an appropriate selection of a potent therapy without actually conducting therapies with trials and errors in diagnosing and treating diseases such as cancers, it is surely expected that such trials will lower patients' physical burdens and the doses of expensive medicines and effectively reduce patients' economical burdens such as medical costs. Although there are not so many actually applicable cases to which the above methods for estimating efficacy of medicines are applicable, future researches would increase such cases. Although there has not yet been established such methods applicable to antitumor agents including TNF-α, such a method, when established, will possibly be a breakthrough in applying to cancer treatment antitumor agents including TNF-α, which could not have been used in clinical treatments because of their undesirable side effects in spite of their outstanding physiological activities.

### Summary of the Invention

The present invention aims to provide a method for estimating the therapeutic efficacy of physiologically active substances or anti-tumor agents, particularly, TNF-α in treating cancers.

To overcome the above object, the present inventors widely screened the expression profiles of genes such as apoptosis-related genes and TNF-α-related genes in established cell lines derived from cancers, found genes which are deeply related to sensitivity to TNF-α; and examined the expression levels of these genes to establish the method for estimating the therapeutic efficacy of TNF-α in cancer treatment. Thus, the present inventors accomplished this invention.

The present invention estimates the therapeutic efficacy of TNF-α in cancer treatment based on the gene expression of TNF-α-related gene, particularly, a protein kinase B (Akt-1) gene, death receptor (DR3) gene, multidrug resistance-associated protein (MRP5) gene, or multidrug resistance-associated protein (MRP6) gene.

### Brief Description of the Accompanying Drawing

FIG. 1 shows a relative expression level of Akt-1 gene with respect to the mRNA level of TNF-α sensitive cells in each rank.

FIG. 2 shows a relative expression level of DR3 gene with respect to the mRNA level of TNF-α sensitive cells in each rank.

FIG. 3 shows a relative expression level of MRP5 gene with respect to the mRNA level of TNF-α sensitive cells in each rank.

FIG. 4 shows a relative expression level of MRP6 gene with respect to the mRNA level of TNF-α sensitive cells in each rank.

FIG. 5 is a comparison of the expression level of Akt-1 gene with respect to mRNA level in each type of cells from different origins.

FIG. 6 is a result of cluster analysis of TNF-α-related gene.

FIG. 7 shows a relationship between the sensitivity of cells to TNF-α and the expression level of Akt-1 gene or ICAM-1, where the symbols "▼" and "○" mean TNF-α-sensitive cells and non-TNF-α-sensitive cells, respectively.

### Detailed Description of the Invention

The term "TNF-α" as referred to as in the present invention means TNF-α in general obtainable from humans or other warm-blooded animals; those produced by culturing cells of humans or other warm-blooded animals in an appropriate manner, and contacting the cells with appropriate TNF-α inducers to produce TNF-α; those produced by preparing appropriate expression vectors introduced with TNF-α genes of humans or other warm-blooded animals, introducing the expression vectors into microorganisms such as *Escherichia coli* or yeasts, animal- or plant-bodies, or cultured animal- or plant-cells, and optionally allowing the resulting transformants to express TNF-α using TNF-α inducers; and those which are totally or partially produced by chemical syntheses by the protein engineering. These TNF-α preparations include natural and recombinant TNF-αs independently of their preparation methods and origins, and further include those which are produced *in vivo* by administering TNF-α inducers to patients or by allowing to express external TNF-α genes introduced into patients' bodies by means of gene therapy. In addition, to control the stability, action and effect of TNF-α, the TNF-αs usable in the present invention include those which are modified with N-glycosylated or O-glycosylated saccharide chains composed of monosaccharides such as glucose, galactose, N-acetyl glucosamine, N-acetyl galactosamine, fucose, mannose, xylose, and sialic acid; those which are modified with saccharide chains composed of saccharides sulfonated with hyaluronic acid or heparan sulfonate; those which are modified with water-soluble high molecules such as polyethylene glycol and poly vinyl alcohol; and those which are partially modified in their amino acid sequences without losing TNF-α activity.

In the present invention, TNF-α can be used in combination with one or more other substances, for example, physiologically active substances such as interferons, interleukins, and growth hormones; and pharmaceuticals such as antitumor agents, antibiotics, vaccines, crude drugs, and herbal medicines.

The term "TNF-α-related genes" as referred to as in the present invention means genes which are induced their expression by TNF-α or which are related to the expression of TNF-α induction, for example, TNF-α-receptor genes, apoptosis-related genes, TNF-α-signal-transduction-related genes, multidrug resistance-associated genes, etc., particularly, the genes in Table 3 as described later. Specifically, prokinase B (hereinafter may be abbreviated as "Akt-1" throughout the specification) gene, a TNF-α-signal-related-gene; death receptor 3 (hereinafter may be abbreviated as "DR3" throughout the specification), receptor-related gene; multidrug resistance-associated protein 5 (hereinafter may be abbreviated as "MRP5" throughout the specification); and multidrug resistance-associated protein 6 (hereinafter may be abbreviated as "MRP6" throughout the specification) which are all deeply related to the therapeutic efficacy of TNF-α because cells, in which these genes are expressed in quantity, have a relatively high sensitivity to TNF-α. Thus, the examination of the expression level of these genes will be advantageously, effectively used in estimating the therapeutic efficacy of TNF-α.

The term "cancer cells" as referred to as in the present invention means those which are derived from cancer tissues, i.e., cancer cells collected from cancer patients or established cell lines, particularly, cancer cells from tissues of cancer patients to be treated are preferable.

The methods used for quantifying the expression level of TNF-α-related genes in the present invention are those for determining the expression level of the genes with respect to the expression level of protein, i.e., those for quantifying the expression level of TNF-α-related proteins using cancer cells intact or after extracting the proteins from the cells with appropriate methods; enzyme immunoassay (EIA), radioimmunoassay (RIA), immunoprecipitation, immunocyte staining method, electrophoresis, western blotting, panning method, high-performance liquid chromatography (HPLC), peptide sequencing method, etc., which can be used in an appropriate combination.

The methods used for determining the expression level of mRNAs of TNF-α-related genes in the present invention are those for quantifying the mRNAs expressed in cancer cells by using the cells intact or by extracting the mRNAs from the cells, or quantifying the level of cDNAs synthesized by reverse transcriptase using the mRNAs as templates; conventional Northern blot technique, Southern blot technique, hybridization method, magnetic bead technology, electrophoresis, polymerase chain reaction (PCR) method, and DNA sequencing method, etc., which can be used in an appropriate combination. In the present invention, the later described real time PCR method as a modified method of PCR method is most preferably used because of its superior processing speed and quantifying accuracy. Without the need of extracting from cancer cells, the mRNAs or their corresponding cDNAs usable in the present invention can be obtainable by appropriately selecting them from RNAs or their corresponding cDNAs, which are derived from commercially available cells, organs, or tissues; as well as their processed products in the form of a pre-blotting membrane or DNA microarray technology.

Explaining the methods used for examining the sensitivity of cancer cells to TNF-α in the present invention, either cancer cells collected from patients suffering from cancers such as intestinal cancer, lung cancer, pancreatic cancer, breast cancer, gastric cancer, hepatoma, renal cancer, neural cancer, skin cancer, cancer of pharynx, sarcoma, and carcinoma uteri; or established cell lines derived from the above cancer cells are cultured in conventional nutrient culture media, supplemented with 0.1 to 10 µg/ml of TNF-α, and incubated for a prescribed period of time, followed by collecting and staining the resulting cells with a dye such as propidium iodide to count death cells or apoptosed cells by a spectrophotometer, flow cytometry, etc. The sensitivity of cells to TNF-α can be determined by comparing the number of death cells or apoptosed cells in the above test cultures with that for a control culture, cultured similarly as in the test cultures but with no addition of TNF-α. Also the sensitivity of cells to TNF-α can be determined by transplanting cancer cells, collected from a cancer patient, to experimental animals such as nude mice, breeding the animals while administering TNF-α and measuring and evaluating the size of the grown tumor masses in the animals.

The methods used for evaluating the expression level of TNF-α-related genes in the present invention include those which calculate the relative expression level of TNF-α-related genes in patients to be administered with TNF-α based on the expression level of genes, as an internal standard, whose expression levels between cells and tissues are not so different, such as glycolytic pathway enzymes such as glyceraldehyde-3-phosphate dehydrogenase or cytoskeleton proteins such as β-actin; those which calculate an increased or decreased level of TNF-α-related genes in patients to be administered with TNF-α based on the expression level of TNF-α-related genes in normal cells of the same patient's tissue as the cancer cells tested. The data on the expression levels thus obtained should preferably be examined whether there exist the desired significant differences under a prescribed level of significant difference by means of Mann-Whitney U test, Student's Welch's t-test, cluster analysis, etc.

The following experiments explain the present invention in detail:

### Experiment 1

### Sensitivity of cell lines, derived from different organs, to TNF-α

Twenty-one different types of cancer cells collected from patients suffering from cancers and 69 different types of established cell lines obtained from American Type Culture Collection (ATCC), Manassas, USA; Japanese Collection of Research Bioresources (JCRB), Tokyo, Japan; European Collection of Cell Cultures (ECACC), North Carolina, USA; German Collection of Microorganisms and Cell Cultures (DSMZ), Braunschweig, Germany; National Institute of Health and Nutrition (NIHN), Tokyo, Japan; and Institute of Development, Aging, and Cancer (IDAC), Miyagi, Japan, were respectively inoculated into an RPMI 1640 medium supplemented with 10% (v/v) fetal calf serum at a cell density of 1 x 10⁴ cells/ml to 3 x 10⁴ cells/ml, cultured for a prescribed period of time, admixed with 5 ng/ml of TNF-α, and incubated at 37°C for 48 hours under 5% (v/v) CO₂ atmospheric conditions. As negative controls, cell culture systems free of TNF-α for each type of cells were provided and cultured similarly as above. After culturing, the cells were collected from the resulting cultures and allowed to stand in a cold 70% (v/v) aqueous ethanol solution for two hours to immobilize the cells. The immobilized cells were stained by keeping in a phosphate-buffered saline (pH 7.2) containing 40 µg/ml of propidium iodide for 20 min. The DNA level in each type of cells was measured on "EPICS XL", a flow cytometry commercialized by Beckman Coulter Inc., CA, USA, and the percentage (%) of apoptosed cells was analyzed on "WINCYCLE", a prescribed software commercialized by Beckman Coulter Inc., CA, USA. The percentages (%) of apoptosed cells for each type of cells treated with TNF-α were minused those which corresponded to negative controls with no TNF-α, and the obtained data were graded into four ranks: A rank which means that it had a percentage (%) of less than 5%; B rank, a percentage (%) of more than 5% but less than 10%; C rank, a percentage (%) of more than 10% but less than 20%; and D rank, a percentage (%) of more than 20%. The results are in Tables 1 and 2.

**Table 1**

| No. | Origin | Cancer cell | | | Percentage (%) of apoptosed cells | Rank |
|---|---|---|---|---|---|---|
| | | Name | Code No. | Obtention | | |
| 1 | Intestinal cancer | From colon cancer patient A | | | 3.7 | A |
| 2 | Intestinal cancer | LoVo | CCL229 | ATCC | 0.1 | A |
| 3 | Intestinal cancer | HCT-15 | CCL225 | ATCC | 28.4 | D |
| 4 | Intestinal cancer | WiDr | CCL218 | ATCC | 5.4 | B |
| 5 | Intestinal cancer | LS174T | CCL188 | ATCC | 7.5 | B |
| 6 | Intestinal cancer | CoLo 205 | CCL222 | ATCC | 29.3 | D |
| 7 | Intestinal cancer | HT-29 | HTB38 | ATCC | 1.6 | A |
| 8 | Intestinal cancer | CoLo 678 | ACC194 | DSMZ | 0.3 | A |
| 9 | Intestinal cancer | SW 1116 | CCL233 | ATCC | 23.8 | D |
| 10 | Intestinal cancer | SW 480 | CCL228 | ATCC | 5.8 | B |
| 11 | Intestinal cancer | CoLo 206 | ACC21 | DSMZ | 10.7 | C |
| 12 | Intestinal cancer | DLD-1 | CCL221 | ATCC | 1.4 | A |
| 13 | Lung cancer | From lung cancer patient A | | | 5.0 | B |
| 14 | Lung cancer | From lung cancer patient B | | | 21.2 | D |
| 15 | Lung cancer | From lung cancer patient C | | | 5.4 | B |
| 16 | Lung cancer | From lung cancer patient D | | | 0.2 | A |
| 17 | Lung cancer | From lung cancer patient E | | | 0.3 | A |
| 18 | Lung cancer | From lung cancer patient F | | | 6.3 | B |
| 19 | Lung cancer | EBC-1 | JCRB0820 | JCRB | 18.6 | C |
| 20 | Lung cancer | MRC-5 | CCL171 | ATCC | 1.2 | A |
| 21 | Lung cancer | CALU6 | HTB56 | ATCC | 1.9 | A |
| 22 | Lung cancer | WI-38VA13 | CCL75.1 | ATCC | 8.3 | B |
| 23 | Lung cancer | OBA-LK-1 | TKG0572 | IDAC | 22.2 | D |
| 24 | Lung cancer | CALU3 | HTB55 | ATCC | 1.7 | A |
| 25 | Lung cancer | A 549 | CCL185 | ATCC | 0.1 | A |
| 26 | Lung cancer | CoLo 699 | ACC196 | DSMZ | 0.8 | A |
| 27 | Lung cancer | LU 65 | JCRB0079 | JCRB | 3.0 | A |
| 28 | Pancreatic cancer | PK-1 | TKG0239 | IDAC | 6.2 | C |
| 29 | Pancreatic cancer | PK-9 | TKG0240 | IDAC | 4.0 | A |
| 30 | Pancreatic cancer | KLM-1 | TKG0490 | IDAC | 1.4 | A |
| 31 | Pancreatic cancer | MIA Paca2 | CRL1420 | ATCC | 7.4 | B |
| 32 | Pancreatic cancer | PANC-1 | CRL1469 | ATCC | 7.6 | B |
| 33 | Pancreatic cancer | PK-59 | TKG0492 | IDAC | 3.0 | A |
| 34 | Pancreatic cancer | PK-8 | TKG0383 | IDAC | 9.6 | B |
| 35 | Pancreatic cancer | PK-45H | TKG0493 | IDAC | 0.6 | A |
| 36 | Breast cancer | ZR-75-1 | CRL1500 | ATCC | 23.4 | D |
| 37 | Breast cancer | YMB-1-E | JCRB0825 | JCRB | 39.4 | D |
| 38 | Breast cancer | MCF-7 | HTB22 | ATCC | 9.5 | B |
| 39 | Gastric cancer | From gastric cancer patient A | | | 2.8 | A |
| 40 | Gastric cancer | MKN-7 | TKG0228 | IDAC | 0.6 | A |
| 41 | Gastric cancer | MKN-74 | JCRB0255 | JCRB | 0.7 | A |
| 42 | Gastric cancer | MKN-45 | JCRB0254 | JCRB | 7.4 | B |
| 43 | Gastric cancer | MKN-1 | JCRB0252 | JCRB | 2.3 | A |
| 44 | Gastric cancer | MKN-28 | JCRB0253 | JCRB | 1.5 | A |
| 45 | Gastric cancer | AZ 521 | JCRB0061 | JCRB | 0.2 | A |
| 46 | Gastric cancer | SCH | JCRB0251 | JCRB | 30.5 | D |
| 47 | Hepatoma | From hepatoma patient A | | | 0.2 | A |
| 48 | Hepatoma | From hepatoma patient B | | | 2.0 | A |
| 49 | Hepatoma | From hepatoma patient C | | | 10.7 | C |
| 50 | Hepatoma | From hepatoma patient D | | | 0.1 | A |
| 51 | Hepatoma | From hepatoma patient E | | | 13.4 | C |
| 52 | Hepatoma | From hepatoma patient F | | | 0.1 | A |
| 53 | Hepatoma | HuH28 | JCRB0426 | JCRB | 0.9 | A |
| 54 | Hepatoma | HuCCT1 | JCRB0425 | JCRB | 0.1 | A |
| 55 | Hepatoma | HuL-1 | | NIHN | 5.8 | B |
| 56 | Hepatoma | HepG2 | HB8065 | ATCC | 2.9 | A |
| 57 | Hepatoma | PLC/PRF/5 | CRL8024 | ATCC | 0.1 | A |
| 58 | Hepatoma | Li-7 | TKG0368 | IDAC | 3.6 | A |
| 59 | Hepatoma | HuH7 | JCRB0403 | JCRB | 1.1 | A |
| 60 | Hepatoma | Hep 3B | HB8064 | ATCC | 3.6 | A |
| 61 | Renal cancer | From renal | cancer | patient A | 3.5 | A |
| 62 | Renal cancer | From renal cancer patient B | | | 0.2 | A |
| 63 | Renal cancer | From renal cancer patient C | | | 5.3 | B |
| 64 | Renal cancer | From renal cancer patient D | | | 0.1 | A |
| 65 | Renal cancer | ACHN | CRL1611 | ATCC | 0.6 | A |
| 66 | Renal cancer | VMRC-RCW | TKG0447 | IDAC | 0.1 | A |
| 67 | Renal cancer | Caki-1 | HTB46 | ATCC | 0.2 | A |
| 68 | Neurologic cancer | From neurologic cancer cancer patient A | | | 0.2 | A |
| 69 | Neurologic cancer | U251 | JRCB0461 | JCRB | 1.0 | A |
| 70 | Neurologic cancer | U373 MG | 89081403 | ECACC | 1.2 | A |
| 71 | Neurologic cancer | SCCH-26 | JCRB0106 | JCRB | 2.7 | A |
| 72 | Neurologic cancer | TN-2 | TKG0278 | IDAC | 1.8 | A |
| 73 | Neurologic cancer | HEPM | CRL1486 | ATCC | 0.1 | A |
| 74 | Neurologic cancer | KINGS-1 | IFO50435 | JCRB | 6.3 | B |
| 75 | Skin cancer | From skin cancer patient A | | | 12.0 | C |
| 76 | Skin cancer | From skin cancer patient B | | | 4.2 | A |
| 77 | Skin cancer | RPMI 7932 | 94072246 | ECACC | 0.1 | A |
| 78 | Skin cancer | G-361 | CRL1424 | ATCC | 0.1 | A |
| 79 | Skin cancer | A375 | CRL1619 | ATCC | 2.8 | A |
| 80 | Skin cancer | SK-MEL-28 | HTB72 | ATCC | 5.9 | B |
| 81 | Throat cancer | Detroit 562 | CCL138 | ATCC | 8.8 | B |
| 82 | Throat cancer | HO-1-u-1 | JCRB0828 | JCRB | 0.4 | A |
| 83 | Throat cancer | HO-1-N-1 | JCRB0831 | JCRB | 3.2 | A |
| 84 | Throat cancer | FADu | HTB43 | ATCC | 0.1 | A |
| 85 | Sarcoma | RD | CCL136 | ATCC | 22.5 | D |
| 86 | Sarcoma | Hu-O9N2 | JCRB0428 | JCRB | 11.6 | C |
| 87 | Sarcoma | Saos-2 | HTB85 | ATCC | 1.3 | A |
| 88 | Carcinoma uteri | Ca Ski | CRL1550 | ATCC | 12.1 | C |
| 89 | Carcinoma uteri | ME-180 | HTB33 | ATCC | 3.0 | A |
| 90 | - | KB | CCL171 | ATCC | 4.7 | A |

As shown in Tables 1 and 2, 56, 18, 7 and 9 out of 90 different types of cells tested were respectively graded into the ranks A to D.

### Experiment 2

### Expression of factors in cells

Using "RNEASY MIDI KIT", an RNA kit commercialized by Qiagen GmbH, Hilden, Germany, RNAs were respectively prepared in usual manner from the 90 different types of cells in Tables 1 and 2. One microgram of each of the RNAs was reacted with 100 µl of a reaction mixture containing one microgram of a separately prepared random hexamer and 100 units of a murine breast viral reverse transcriptase sequentially at 25 C for 10 min, at 42°C for 30 min, and 99°C for 5 min, and then the reaction was suspended to obtain a cDNA.

Primers for PCR in SEQ ID NOs: 1 to 100, which corresponded to 49 different types of TNF-α-related genes, registered at Unigene, a DNA database; and to a β-actin gene as an internal standard gene, were prepared and in usual manner subjected to real time PCR using "ABIPRISM 7700 SEQUENCE DETECTION SYSTEM", an apparatus for PCR commercialized by Applied Biosystems Japan, Ltd., Tokyo, Japan: Twenty nanograms of a cDNA, 100 nM of a sense primer, and 100 nM of an antisense primer were dissolved in water to obtain a 12.5 µl aqueous solution which was then admixed with 12.5 µl of "SYBR GREEN PCR MASTERMIX" commercialized by Applied Biosystems Japan, Inc., Tokyo, Japan, to obtain a reaction mixture. Then, the reaction mixture was subjected to 45 cycles of PCR with sequential incubations at 90°C for 15 sec and at 60°C for one min, followed by comparing the expression levels for each gene with that of the internal (3-actin gene to express the levels in a numerical manner as their relative expression levels. According to the grading of the ranks in Experiment 1, the ranks B, C and D against the rank A in each gene were examined according to Mann-Whitney U test, and the results were evaluated whether they had a significant difference at p<0.01 or p<0.05. The results are in Table 3.

**Table 3**

| No. | Gene | Group | | | No. | Gene | Group | | |
|---|---|---|---|---|---|---|---|---|---|
| | | B | C | D | | | B | C | D |
| 1 | TNF-R55 | + | - | - | 26 | NF-kBp65 | - | - | - |
| 2 | TNF-R75 | - | - | - | 27 | N-SMase | - | - | - |
| 3 | TIMP2 | - | - | - | 28 | ERK1 | - | - | - |
| 4 | SODD | - | - | - | 29 | ERK2 | - | - | - |
| 5 | TACE | - | - | - | 30 | p38 | - | - | - |
| 6 | TNF-α | - | - | - | 31 | CyclinG1 | - | - | - |
| 7 | TRAF1 | + | + | - | 32 | apoptosis | - | - | - |
| 8 | TRAF2 | - | - | - | | inhibitor 4 | | | |
| 9 | FAN | - | - | - | 33 | cdc25b | - | - | - |
| 10 | Caspase-8 | - | - | - | 34 | CyclinD1 | - | - | - |
| 11 | Caspase-3 | - | - | - | 35 | JAB | - | - | - |
| 12 | Caspase-9 | - | - | - | 36 | PKR | - | - | - |
| 13 | HIAP1 | - | - | - | 37 | 2,5-0A | - | - | - |
| 14 | HIAP2 | - | - | - | 38 | MDR1 | - | - | - |
| 15 | Akt-1 | + | + | ++ | 39 | MDR3 | - | - | - |
| 16 | Bcl-1 | - | - | - | 40 | MRP1 | - | - | - |
| 17 | Bcl-xL | - | - | - | 41 | MRP2 | - | - | - |
| 18 | BAD | - | - | - | 42 | MRP3 | - | - | - |
| 19 | Bax | - | - | - | 43 | MRP4 | - | - | - |
| 20 | p53 | - | - | + | 44 | MRP5 | + | + | + |
| 21 | Mn-SOD | - | - | - | 45 | MRP6 | - | + | ++ |
| 22 | IKK1 | - | - | - | 46 | BCRP | - | - | - |
| 23 | DR3 | + | + | + | 47 | COX-2 | - | - | - |
| 24 | PKC-β1 | - | - | - | 48 | iNOS | - | - | - |
| 25 | NF-kBp50 | - | - | - | 49 | TGF-β | - | - | - |
| "-" : It had no significant difference compared with the group A. | | | | | | | | | |
| "+" : It had a significant difference (p<0.05) compared with the group A. | | | | | | | | | |
| "++" : It had a significant difference (p<0.01) compared with the group A. | | | | | | | | | |

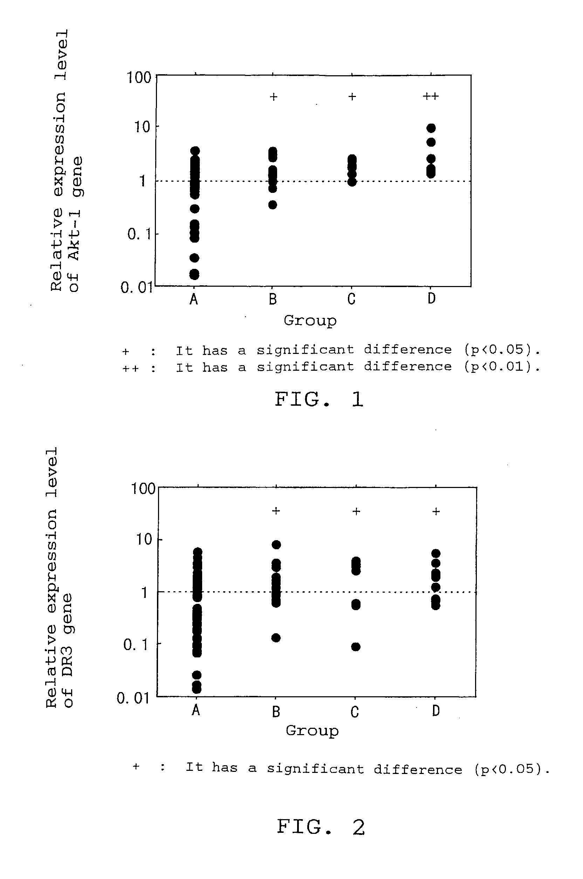

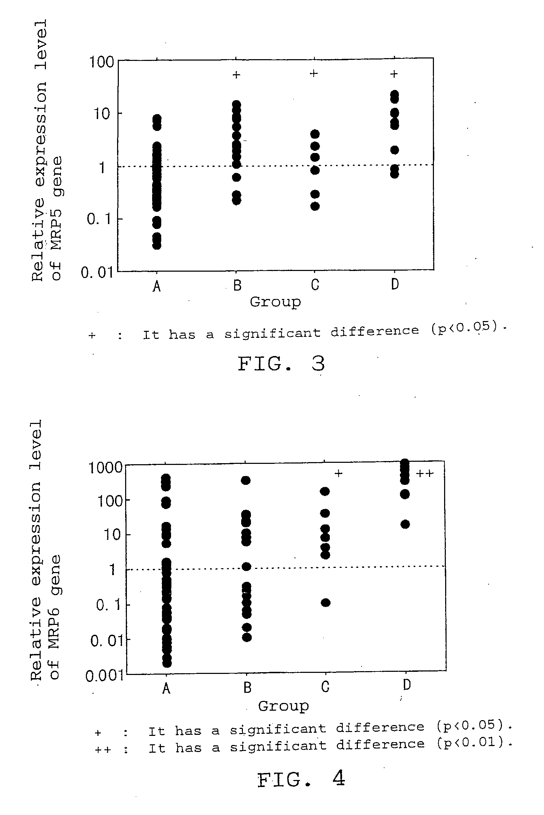

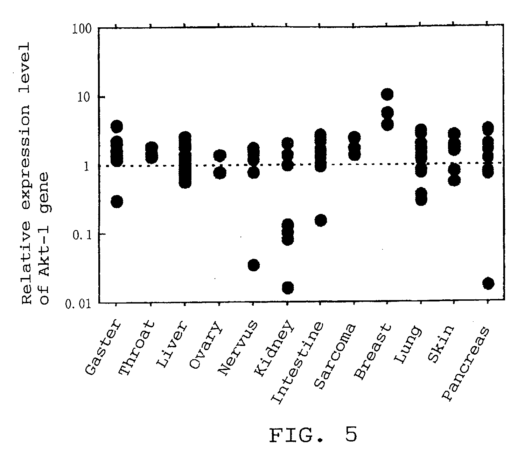

As evident from Table 3, the expression levels of Akt-1, DR3, MRP5 and MRP6 genes with respect to the mRNA levels in the cells with a relatively high sensitivity to TNF-α showed a statistically significantly high expression level. FIGs. 1 to 4 show the results of statistical works of all the above expression levels. As shown in FIG.5, the data on the expression level of Akt-1 gene with respect to the mRNA level in respective established cell lines from intestinal, breast, lung, and pancreatic organs revealed that the cell lines had a higher expression level of Akt-1 gene, resulting in an estimation that the cancers in such organs would have a high potential of being cured by TNF-α.

### Experiment 3

### Cluster analysis

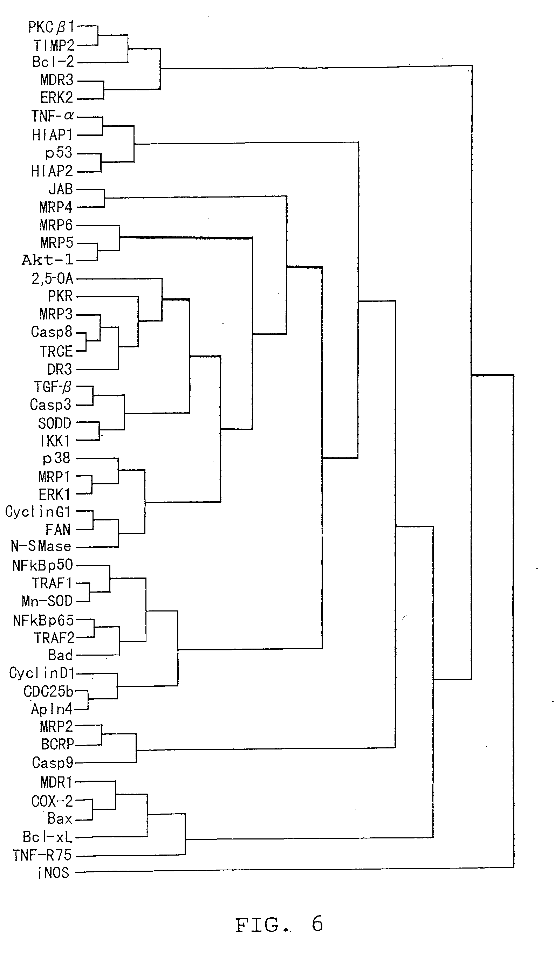

Based on the profiles of the 49 different types of genes as the results in Experiment 2, the cluster analysis between the genes was conducted by conventional analysis for factors and main ingredients using "EPCLUST", a commercially available computer software of European Bioinfomatics Institute, Cambridge, UK. The results are in FIG. 6, revealing that Akt-1, MRP5 and MRP6 genes can be classified into the same cluster which exhibits substantially the same dynamics. The data indicates that there exist at least two representative groups of Akt-1 and DR3 genes as gene groups which enable the estimation of therapeutic efficacy of TNF-α.

### Experiment 4

### Expression of cell membrane antigen

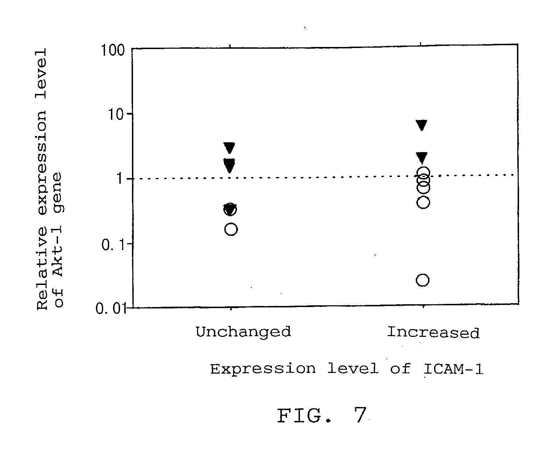

In addition to the action of inducing cells to death through the induction of apoptosis, TNF-α has also the action of promoting the expression of ICAM-1 as a cell membrane antigen. It was examined whether there exists any correlation between the expressions of Akt-1 gene and ICAM-1: With reference to the results in Experiment 1, seven types of TNF-α sensitive cells, i.e., those from the lung cancer patient B, HCT-15 cells, RD cells, OBA-LK-1 cells, CoLo 205 cells, CoLo 206 cells, a YMB-1-E cells; and seven types of non-TNF-α-sensitive cells, i.e., those from the colon cancer patient A, the skin cancer patient A, the lung cancer patient E, the neurologic cancer patient A, the hepatoma patient D, PK-45H cells, and MKN-7 cells were selected, and each of which was suspended in an RPMI 1640 medium supplemented with 10% (v/v) of fetal calf serum to give a cell density of 2x10⁴ cells/ml. To three milliliters of each of the resulting cell suspensions, placed in a commercialized 6-well-plate, was added 5 ng/ml of a human TNF-α preparation, and the cell suspensions were incubated at 37°C for 24 hours under 5% CO₂ atmospheric conditions. After culturing, the cells were collected from each culture, and then successively treated with "BBA-3", a murine anti-human ICAM-1 antibody, commercialized by R & D Systems Inc., Minneapolis, USA, at 4°C for 40 min, successively washed with a phosphate buffered saline containing 1% (v/v) of calf serum albumin, a fluorescein-labelled anti-mouse immunoglobulin G antibody at 4°C for 40 min, and 3% (v/v) formalin. The resulting cells were measured for fluorescent intensity on "EPICS XL", a flow cytometry commercialized by Beckman Coulter Inc., CA, USA, to examine the expression level of ICAM-1 on the surface of cell membranes. The relationship between the data from the flow cytometry and the expression data of Akt-1 gene in Experiment 2 was studied. The results are in FIG. 7. In FIG. 7, the symbols "▼" and "○" mean TNF-α-sensitive cells and non-TNF-α-sensitive cells, respectively.

As found in FIG. 7, two out of the seven different types of TNF-a-sensitive cells ("▼") increased in the expression level of ICAM-1 but the resting five types of cells did not show any change in the expression level, while two out of the seven different types of non-TNF-α-sensitive cells ("○") did not exhibit any change in the expression level of ICAM-1 but the resting five types of cells increased in the expression level, and this gave no statistically significant relationship between the TNF-α sensitivity and the expression level of ICAM-1. The TNF-α-sensitive cells "▼" was significantly high in the expression level of Akt-1 gene with respect to its corresponding mRNA level, and there was found a relatively high correlation between the TNF-α-sensitivity and the expression level of Akt-1 gene when examined on Mann-Whitney U test. These results show that the estimation of the therapeutic efficacy of TNF-α by examining the expression level of Akt-1 gene is more preferable in estimating the antitumor effect of TNF-α through the induction of apoptosis than by estimating the expression level of Akt-1 gene as one of the various functions and effects of TNF-α.

The following example explain the present invention in detail, but should not limit the present invention:

### Example

Cancer cells, extracted from five patients suffering from lung cancer, were treated in usual manner to collect RNAs. Using the RNAs as templates, their corresponding cDNAs were prepared with a murine breast viral reverse transcriptase. A reaction mixture was prepared by adding to 20 µg of each of the cDNAs as templates 12.5 µg of "SYBR GREEN PCR MASTERMIX" containing a thermostable DNA polymerase etc., commercialized by Applied Biosystems Japan, Inc., Tokyo, Japan, and either 100 nM of primers for detection of Akt-1 mRNA having nucleotide sequences of SEQ ID NOs:29 and 30, or 100 nM of primers for detection of β-actin mRNA having nucleotide sequences of SEQ ID NOs:99 and 100. The resulting reaction mixtures were subjected to 45 cycles of PCR sequentially at 90°C for 15 sec and at 60°C for one minute using "ABIPRISM 7700 SEQUENCE DETECTION SYSTEM", a real time PCR apparatus. With reference to the expression level of β-actin mRNA as an internal standard, the expression level of Akt-1 gene was calculated.

The cells from each of the above five patients suffering from lung cancer were intraperitoneally transplanted to 10 nude mice at a cell density of 1 x 10⁴ cells/head. On two days after the transplantation, five nude mice out of the 10 mice were intravenously administered with 5 ng/head of TNF-α. On three days after the administration, the tumor masses in the mice were measured according to the method disclosed by K. Nakahara in *"International Journal of Medicine"*, Vol. 34, pp. 263-267 (1984) and compared with those of control nude mice with no administration of TNF-α to evaluate the antitumor effect of TNF-α. As a result, the more the expression level of Akt-1 gene with respect to mRNA level the higher the antitumor effect of TNF-α.

As described above, according to the present invention, the therapeutic efficacy of TNF-α on cancer cells will be estimated by measuring the expression level of genes such as of Akt-1, DR3, etc., because the sensitivity of TNF-α on cancer cells highly correlates with the expression level of such genes. Thus, the application of the present invention to patients prior to the administration of TNF-α, the desired therapeutic efficacy of TNF-α will be estimated in a safe and sure manner. The measurement of the expression level of Akt-1 gene is suitable for estimating the antitumor effect of TNF-α among the actions and functions of TNF-α. Thus, the present invention enables the screening of the possibility of pharmaceutical uses of TNF-α which the uses had been deemed actually impossible. In addition, the present invention can be applied to examination of the types of cancers treatable with antitumor agents such as TNF-α.

While there has been described what is at present considered to be the preferred embodiments of the invention, it will be understood the various modifications may be made therein, and it is intended to cover in the appended claims all such modifications as fall within the true spirits and scope of the invention.

## Claims

1. A method for estimating the therapeutic efficacy of tumor necrosis factor, comprising a step of evaluating the expression level of a tumor necrosis factor-related gene in a cancer cell.

2. The method of claim 1, wherein said tumor necrosis factor-related gene is one or more genes selected from the group consisting of a protein kinase B (Akt-1) gene, death receptor (DR3) gene, multidrug resistance-associated protein (MRP5) gene, and multidrug resistance-associated protein (MRP6) gene.

3. The method of claim 1, wherein said cancer cell is an established cell or a cancer cell derived from a cancer patient.

4. A kit for estimating the therapeutic efficacy of tumor necrosis factor used in the treatment of cancers, which comprises a thermostable DNA polymerase and an oligonucleotide primer comprising a DNA sequence encoding a gene selected from the group consisitng of a protein kinase B (Akt-1) gene, death receptor (DR3) gene, multidrug resistance-associated protein (MRP5) gene, and multidrug resistance-associated protein (MRP6) gene.


SEQUENCE LISTING

<110> Kabushiki Kaisha Hayashibara Seibutsu Kagaku Kenkyujo
<120> Method for estimating therapeutic efficacy of tumor
necrosis factor
<130> 303317EP/JND/AV/ms
<150> JP 107126/2002
<151> 2002-4-9
<160> 100

<200>
<210> 1
<211> 22
<212> DNA
<213> Artificial sequence
<220>
<223> Oligonucleotide used as primer for PCR detection of TNF-R55
mRNA

<300>
<308> Unigene Hs.159

<400> 1
cctgccagga gaaacagaac ac

<200>
<210> 2
<211> 21
<212> DNA
<213> Artificial sequence
<220>
<223> Oligonucleotide used as primer for PCR detection of TNF-R55
mRNA

<300>
<308> Unigene Hs.159

<400> 2
gggactgaag ctttgggtttg g

<200>
<210> 3
<211> 22
<212> DNA
<213> Artificial sequence
<220>
<223> Oligonucleotide used as primer for PCR detection of TNF-R75
mRNA

<300>
<308> Unigene Hs.256278

<400> 3
gccccaccag atctgtaacg tg

<200>
<210> 4
<211> 21
<212> DNA
<213> Artificial sequence
<220>
<223> Oligonucleotide used as primer for PCR detection of TNF-R75
mRNA

<300>
<308> Unigene Hs.256278

<400> 4
tgaggcacct tggcttctct c

<200>
<210> 5

<211> 22
<212> DNA
<213> Artificial sequence
<220>
<223> Oligonucleotide used as primer for PCR detection of TIMP2
mRNA

<300>
<308> Unigene Hs.325495

<400> 5
gcggtcagtg agaaggaagt gg

<200>
<210> 6
<211> 23
<212> DNA
<213> Artificial sequence
<220>
<223> Oligonucleotide used as primer for PCR detection of TIMP2
mRNA

<300>
<308> Unigene Hs.325495

<400> 6
ggagatgtag cacgggatca tgg

<200>
<210> 7
<211> 20
<212> DNA
<213> Artificial sequence
<220>
<223> Oligonucleotide used as primer for PCR detection of SODD mRNA

<300>
<308> Unigene Hs.194726

<400> 7
acagcccaac tccagtctct

<200>
<210> 8
<211> 20
<212> DNA
<213> Artificial sequence
<220>
<223> Oligonucleotide used as primer for PCR detection of SODD mRNA

<300>
<308> Unigene Hs.194726

<400> 8
aagttgtgcc ggttcatgct

<200>
<210> 9
<211> 22
<212> DNA
<213> Artificial sequence
<220>
<223> Oligonucleotide used as primer for PCR detection of
TACE(ADAM17) mRNA

<300>
<308> Unigene Hs. 4311

<400> 9
ctgcacaggt aatagcagtg ag

<200>

<210> 10
<211> 22
<212> DNA
<213> Artificial sequence
<220>
<223> Oligonucleotide used as primer for PCR detection of
TACE(ADAM17) mRNA

<300>
<308> Unigene Hs.64311

<400> 10
ctcagctggt caatgaaatc cc

<200>
<210> 11
<211> 22
<212> DNA
<213> Artificial sequence
<220>
<223> Oligonucleotide used as primer for PCR detection of TNF-alpha
mRNA

<300>
<308> Unigene Hs.241570

<400> 11
ttctcgaacc ccgagtgaca ag

<200>
<210> 12
<211> 21
<212> DNA
<213> Artificial sequence
<220>
<223> Oligonucleotide used as primer for PCR detection of TNF-alpha

mRNA

<300>
<308> Unigene Hs.241570

<400> 12
cccttctcca gctggaagac c

<200>
<210> 13
<211> 23
<212> DNA
<213> Artificial sequence
<220>
<223> Oligonucleotide used as primer for PCR detection of TRAF1
mRNA

<300>
<308> Unigene Hs.2134

<400> 13
gcactttcct gtggaagatc acc

<200>
<210> 14
<211> 21
<212> DNA
<213> Artificial sequence
<220>
<223> Oligonucleotide used as primer for PCR detection of TRAF1
mRNA

<300>
<308> Unigene Hs.2134

<400> 14

ctggccacgt tggtttcact c

<200>
<210> 15
<211> 22
<212> DNA
<213> Artificial sequence
<220>
<223> Oligonucleotide used as primer for PCR detection of TRAF2
mRNA

<300>
<308> Unigene Hs.200526

<400> 15
aacattgtct gcgtcctgaa cc

<200>
<210> 16
<211> 24
<212> DNA
<213> Artificial sequence
<220>
<223> Oligonucleotide used as primer for PCR detection of TRAF2
mRNA

<300>
<308> Unigene Hs.200526

<400> 16
cgttcaggta gatacgcaga caca

<200>
<210> 17
<211> 19
<212> DNA

<213> Artificial sequence
<220>
<223> Oligonucleotide used as primer for PCR detection of FAN mRNA

<300>
<308> Unigene Hs.78687

<400> 17
tgcctctggg cttggaagt

<200>
<210> 18
<211> 21
<212> DNA
<213> Artificial sequence
<220>
<223> Oligonucleotide used as primer for PCR detection of FAN mRNA

<300>
<308> Unigene Hs.78687

<400> 18
tcaggcattt cctggtagcg t

<200>
<210> 19
<211> 21
<212> DNA
<213> Artificial sequence
<220>
<223> Oligonucleotide used as primer for PCR detection of Caspase-8
mRNA

<300>
<308> Unigene Hs.19949

<400> 19
cacgggagaa agtgcccaaa c

<200>
<210> 20
<211> 24
<212> DNA
<213> Artificial sequence
<220>
<223> Oligonucleotide used as primer for PCR detection of Caspase-8
mRNA

<300>
<308> Unigene Hs.19949

<400> 20
ggttgctcct ctgaatcagt ctca

<200>
<210> 21
<211> 21
<212> DNA
<213> Artificial sequence
<220>
<223> Oligonucleotide used as primer for PCR detection of Caspase-3
mRNA

<300>
<308> Unigene Hs.74552

<400> 21
gctgtctacg gcacatggtg a

<200>
<210> 22
<211> 22

<212> DNA
<213> Artificial sequence
<220>
<223> Oligonucleotide used as primer for PCR detection of Caspase-3
mRNA

<300>
<308> Unigene Hs.74552

<400> 22
gttgccacct ttcggttaac cc

<200>
<210> 23
<211> 20
<212> DNA
<213> Artificial sequence
<220>
<223> Oligonucleotide used as primer for PCR detection of Caspase-9
mRNA

<300>
<308> Unigene Hs.100641

<400> 23
gctgtctacg gcacagatgg

<200>
<210> 24
<211> 21
<212> DNA
<213> Artificial sequence
<220>
<223> Oligonucleotide used as primer for PCR detection of Caspase-9
mRNA

<300>
<308> Unigene Hs.100641

<400> 24
gatgtcgtcc agggtctcaa c

<200>
<210> 25
<211> 24
<212> DNA
<213> Artificial sequence
<220>
<223> Oligonucleotide used as primer for PCR detection of HIAP1
mRNA

<300>
<308> Unigene Hs.127799

<400> 25
actacatagg acctggagac agag

<200>
<210> 26
<211> 24
<212> DNA
<213> Artificial sequence
<220>
<223> Oligonucleotide used as primer for PCR detection of HIAP1
mRNA

<300>
<308> Unigene Hs.127799

<400> 26
aagtactcac accttggaaa ccac

<200>
<210> 27
<211> 24
<212> DNA
<213> Artificial sequence
<220>
<223> Oligonucleotide used as primer for PCR detection of HIAP2
mRNA

<300>
<308> Unigene Hs.289107

<400> 27
tcagtaactg ggaaccaaag gatg

<200>
<210> 28
<211> 24
<212> DNA
<213> Artificial sequence
<220>
<223> Oligonucleotide used as primer for PCR detection of HIAP2
mRNA

<300>
<308> Unigene Hs.289107

<400> 28
aagtactcac accttggaaa ccac

<200>
<210> 29
<211> 20
<212> DNA
<213> Artificial sequence
<220>

<223> Oligonucleotide used as primer for PCR detection of Akt-1
mRNA

<300>
<308> Unigene Hs.71816

<400> 29
tgtgtcagcc ctggactacc

<200>
<210> 30
<211> 20
<212> DNA
<213> Artificial sequence
<220>
<223> Oligonucleotide used as primer for PCR detection of Akt-1
mRNA

<300>
<308> Unigene Hs.71816

<400> 30
tgagcagccc tgaaagcaag

<200>
<210> 31
<211> 19
<212> DNA
<213> Artificial sequence
<220>
<223> Oligonucleotide used as primer for PCR detection of Bcl-2
mRNA

<300>
<308> Unigene Hs.79241

<400> 31
tgcacctgac gcccttcac

<200>
<210> 32
<211> 21
<212> DNA
<213> Artificial sequence
<220>
<223> Oligonucleotide used as primer for PCR detection of Bcl-2
mRNA

<300>
<308> Unigene Hs.79241

<400> 32
cacttgtggc ccagataggc a

<200>
<210> 33
<211> 22
<212> DNA
<213> Artificial sequence
<220>
<223> Oligonucleotide used as primer for PCR detection of Bcl-XL
mRNA

<300>
<308> Unigene Hs.305890

<400> 33
aagggactga atcggagatg ga

<200>
<210> 34
<211> 19

<212> DNA
<213> Artificial sequence
<220>
<223> Oligonucleotide used as primer for PCR detection of Bcl-XL
mRNA

<300>
<308> Unigene Hs.305890

<400> 34
catgcccgtc aggaaccag

<200>
<210> 35
<211> 21
<212> DNA
<213> Artificial sequence
<220>
<223> Oligonucleotide used as primer for PCR detection of BAD mRNA

<300>
<308> Unigene Hs.76366

<400> 35
cgagtgagca ggaagactcc a

<200>
<210> 36
<211> 22
<212> DNA
<213> Artificial sequence
<220>
<223> Oligonucleotide used as primer for PCR detection of BAD mRNA

<300>
<308> Unigene Hs.76366

<400> 36
aggagtccac aaactcgtca ct

<200>
<210> 37
<211> 21
<212> DNA
<213> Artificial sequence
<220>
<223> Oligonucleotide used as primer for PCR detection of Bax mRNA

<300>
<308> Unigene Hs.159428

<400> 37
gagctgcaga ggatgattgc c

<200>
<210> 38
<211> 20
<212> DNA
<213> Artificial sequence
<220>
<223> Oligonucleotide used as primer for PCR detection of Bax mRNA

<300>
<308> Unigene Hs.159428

<400> 38
ccactgtgac ctgctccaga

<200>
<210> 39
<211> 20
<212> DNA

<213> Artificial sequence
<220>
<223> Oligonucleotide used as primer for PCR detection of p53 mRNA

<300>
<308> Unigene Hs.1846

<400> 39
tgcagctgtg ggttgattcc

<200>
<210> 40
<211> 22
<212> DNA
<213> Artificial sequence
<220>
<223> Oligonucleotide used as primer for PCR detection of p53 mRNA

<300>
<308> Unigene Hs.1846

<400> 40
aacacgcacc tcaaagctgt tc

<200>
<210> 41
<211> 22
<212> DNA
<213> Artificial sequence
<220>
<223> Oligonucleotide used as primer for PCR detection of Mn-SOD
mRNA

<300>
<308> Unigene Hs.318885

<400> 41
aacgtcaccg aggagaagta cc

<200>
<210> 42
<211> 22
<212> DNA
<213> Artificial sequence
<220>
<223> Oligonucleotide used as primer for PCR detection of Mn-SOD
mRNA

<300>
<308> Unigene Hs.318885

<400> 42
cagcagtgga ataaggcctg tt

<200>
<210> 43
<211> 21
<212> DNA
<213> Artificial sequence
<220>
<223> Oligonucleotide used as primer for PCR detection of IKK1 mRNA

<300>
<308> Unigene Hs.198998

<400> 43
gcgagcagat gacgtatggg a

<200>
<210> 44
<211> 22
<212> DNA

<213> Artificial sequence
<220>
<223> Oligonucleotide used as primer for PCR detection of IKK1 mRNA

<300>
<308> Unigene Hs.198998

<400> 44
gcttacagcc caacaacttg ct

<200>
<210> 45
<211> 22
<212> DNA
<213> Artificial sequence
<220>
<223> Oligonucleotide used as primer for PCR detection of DR3 mRNA

<300>
<308> Unigene Hs.180338

<400> 45
actgccaacc atgcctagac tg

<200>
<210> 46
<211> 20
<212> DNA
<213> Artificial sequence
<220>
<223> Oligonucleotide used as primer for PCR detection of DR3 mRNA

<300>
<308> Unigene Hs.180338

<400> 46

agagcctcca tcccagcttc

<200>
<210> 47
<211> 22
<212> DNA
<213> Artificial sequence
<220>
<223> Oligonucleotide used as primer for PCR detection of PKC-beta1
mRNA

<300>
<308> Unigene Hs.77202

<400> 47
tgagagggcc aagatcagtc ag

<200>
<210> 48
<211> 20
<212> DNA
<213> Artificial sequence
<220>
<223> Oligonucleotide used as primer for PCR detection of PKC-beta1
mRNA

<300>
<308> Unigene Hs.77202

<400> 48
agtacaggcg gtccatggtc

<200>
<210> 49
<211> 20
<212> DNA

<213> Artificial sequence
<220>
<223> Oligonucleotide used as primer for PCR detection of NF-[?]Bp50
mRNA

<300>
<308> Unigene Hs.73090

<400> 49
gcagcgagcc attgcctttc

<200>
<210> 50
<211> 22
<212> DNA
<213> Artificial sequence
<220>
<223> Oligonucleotide used as primer for PCR detection of NF-[?]Bp50
mRNA

<300>
<308> Unigene Hs.73090

<400> 50
ggtccagcat ggtgaagagt gt

<200>
<210> 51
<211> 21
<212> DNA
<213> Artificial sequence
<220>
<223> Oligonucleotide used as primer for PCR detection of NF-[?]Bp65
mRNA

<300>

<308> Unigene Hs.75569

<400> 51
ctgatgtgca ccgacaagtg g

<200>
<210> 52
<211> 22
<212> DNA
<213> Artificial sequence
<220>
<223> Oligonucleotide used as primer for PCR detection of NF-□Bp65
mRNA

<300>
<308> Unigene Hs.75569

<400> 52
gttgatggtg ctcagggatg ac

<200>
<210> 53
<211> 22
<212> DNA
<213> Artificial sequence
<220>
<223> Oligonucleotide used as primer for PCR detection of N-SMase
mRNA

<300>
<308> Unigene Hs.55235

<400> 53
ggcctctgtg tcttctccaa ac

<200>

<210> 54
<211> 20
<212> DNA
<213> Artificial sequence
<220>
<223> Oligonucleotide used as primer for PCR detection of N-SMase
mRNA

<300>
<308> Unigene Hs.55235

<400> 54
aagccctgtc cactccttca

<200>
<210> 55
<211> 22
<212> DNA
<213> Artificial sequence
<220>
<223> Oligonucleotide used as primer for PCR detection of ERK1 mRNA

<300>
<308> Unigene Hs.861

<400> 55
gcaggacctg atggagactg ac

<200>
<210> 56
<211> 20
<212> DNA
<213> Artificial sequence
<220>
<223> Oligonucleotide used as primer for PCR detection of ERK1 mRNA

<300>
<308> Unigene Hs.861

<400> 56
ccagaatgca gcccacagac

<200>
<210> 57
<211> 20
<212> DNA
<213> Artificial sequence
<220>
<223> Oligonucleotide used as primer for PCR detection of ERK2 mRNA

<300>
<308> Unigene Hs.24473

<400> 57
gcgctacacc aacctctcgt

<200>
<210> 58
<211> 21
<212> DNA
<213> Artificial sequence
<220>
<223> Oligonucleotide used as primer for PCR detection of ERK2 mRNA

<300>
<308> Unigene Hs.324473

<400> 58
cacggtgcag aacgttagct g

<200>
<210> 59

<211> 20
<212> DNA
<213> Artificial sequence
<220>
<223> Oligonucleotide used as primer for PCR detection of p38 mRNA

<300>
<308> Unigene Hs.79107

<400> 59
gccgagctgt tgactggaag

<200>
<210> 60
<211> 21
<212> DNA
<213> Artificial sequence
<220>
<223> Oligonucleotide used as primer for PCR detection of p38 mRNA

<300>
<308> Unigene Hs.79107

<400> 60
ggaggtccct gctttcaaag g

<200>
<210> 61
<211> 22
<212> DNA
<213> Artificial sequence
<220>
<223> Oligonucleotide used as primer for PCR detection of cyclin G1
mRNA

<300>

<308> Unigene Hs.79101

<400> 61
ttggcaactg acttgatccg aa

<200>
<210> 62
<211> 21
<212> DNA
<213> Artificial sequence
<220>
<223> Oligonucleotide used as primer for PCR detection of cyclin G1
mRNA

<300>
<308> Unigene Hs.79101

<400> 62
caagctcttg ccagaaggtc a

<200>
<210> 63
<211> 20
<212> DNA
<213> Artificial sequence
<220>
<223> Oligonucleotide used as primer for PCR detection of apoptosis
inhibitor 4 mRNA

<300>
<308> Unigene Hs.1578

<400> 63
cgaggctggc ttcatccact

<200>

<210> 64
<211> 19
<212> DNA
<213> Artificial sequence
<220>
<223> Oligonucleotide used as primer for PCR detection of apoptosis
inhibitor 4 mRNA

<300>
<308> Unigene Hs.1578

<400> 64
acggcgcact ttcttcgca

<200>
<210> 65
<211> 22
<212> DNA
<213> Artificial sequence
<220>
<223> Oligonucleotide used as primer for PCR detection of cdc25B
mRNA

<300>
<308> Unigene Hs.153752

<400> 65
gctctgggga agacaaggag aa

<200>
<210> 66
<211> 22
<212> DNA
<213> Artificial sequence
<220>
<223> Oligonucleotide used as primer for PCR detection of cdc25B

mRNA

<300>
<308> Unigene Hs.153752

<400> 66
tggcacttgc tgtacatgac ga

<200>
<210> 67
<211> 19
<212> DNA
<213> Artificial sequence
<220>
<223> Oligonucleotide used as primer for PCR detection of cyclin D1
mRNA

<300>
<308> Unigene Hs.82932

<400> 67
acgaaggtct gcgcgtgtt

<200>
<210> 68
<211> 20
<212> DNA
<213> Artificial sequence
<220>
<223> Oligonucleotide used as primer for PCR detection of cyclin D1
mRNA

<300>
<308> Unigene Hs.82932

<400> 68

ccgctggcca tgaactgcct

<200>
<210> 69
<211> 20
<212> DNA
<213> Artificial sequence
<220>
<223> Oligonucleotide used as primer for PCR detection of JAB mRNA

<300>
<308> Unigene Hs.50640

<400> 69
gtggcagccg acaatgcagt

<200>
<210> 70
<211> 22
<212> DNA
<213> Artificial sequence
<220>
<223> Oligonucleotide used as primer for PCR detection of JAB mRNA

<300>
<308> Unigene Hs.50640

<400> 70
cgaggccatc ttcacgctaa gg

<200>
<210> 71
<211> 22
<212> DNA
<213> Artificial sequence
<220>

<223> Oligonucleotide used as primer for PCR detection of PKR mRNA

<300>
<308> Unigene Hs.30376

<400> 71
cgcagccaaa ttagctgttg ag

<200>
<210> 72
<211> 22
<212> DNA
<213> Artificial sequence
<220>
<223> Oligonucleotide used as primer for PCR detection of PKR mRNA

<300>
<308> Unigene Hs.30376

<400> 72
ttgctttggg actcacacgt ag

<200>
<210> 73
<211> 20
<212> DNA
<213> Artificial sequence
<220>
<223> Oligonucleotide used as primer for PCR detection of 2',5'-OAS
mRNA

<300>
<308> Unigene Hs.118633

<400> 73
cgtgcgctca gcttcgtact

<200>
<210> 74
<211> 21
<212> DNA
<213> Artificial sequence
<220>
<223> Oligonucleotide used as primer for PCR detection of 2',5'-OAS
mRNA

<300>
<308> Unigene Hs.118633

<400> 74
tactgaggtg gcagcttccc a

<200>
<210> 75
<211> 21
<212> DNA
<213> Artificial sequence
<220>
<223> Oligonucleotide used as primer for PCR detection of MDR1 mRNA

<300>
<308> Unigene Hs.21330

<400> 75
tagcggctct tccaagctca a

<200>
<210> 76
<211> 22
<212> DNA
<213> Artificial sequence
<220>

<223> Oligonucleotide used as primer for PCR detection of MDR1 mRNA

<300>
<308> Unigene Hs.21330

<400> 76
caacatggtc cagtgccact ac

<200>
<210> 77
<211> 21
<212> DNA
<213> Artificial sequence
<220>
<223> Oligonucleotide used as primer for PCR detection of MDR3 mRNA

<300>
<308> Unigene Hs.73812

<400> 77
tggccctggt tggaagtagt g

<200>
<210> 78
<211> 22
<212> DNA
<213> Artificial sequence
<220>
<223> Oligonucleotide used as primer for PCR detection of MDR3 mRNA

<300>
<308> Unigene Hs.73812

<400> 78
agaaggatct tggggttgcg aa

<200>
<210> 79
<211> 22
<212> DNA
<213> Artificial sequence
<220>
<223> Oligonucleotide used as primer for PCR detection of MRP1 mRNA

<300>
<308> Unigene Hs.89433

<400> 79
cggaaaccat ccacgaccct aa

<200>
<210> 80
<211> 22
<212> DNA
<213> Artificial sequence
<220>
<223> Oligonucleotide used as primer for PCR detection of MRP1 mRNA

<300>
<308> Unigene Hs.89433

<400> 80
tcatgaggaa gtagggccca aa

<200>
<210> 81
<211> 20
<212> DNA
<213> Artificial sequence
<220>
<223> Oligonucleotide used as primer for PCR detection of MRP2 mRNA

<300>
<308> Unigene Hs.193852

<400> 81
gtcctggctg gagtcgcttt

<200>
<210> 82
<211> 21
<212> DNA
<213> Artificial sequence
<220>
<223> Oligonucleotide used as primer for PCR detection of MRP2 mRNA

<300>
<308> Unigene Hs.193852

<400> 82
ggcgtccagc acattgtttg g

<200>
<210> 83
<211> 19
<212> DNA
<213> Artificial sequence
<220>
<223> Oligonucleotide used as primer for PCR detection of MRP3 mRNA

<300>
<308> Unigene Hs.90786

<400> 83
tcctggctgg agtcgcttt

<200>
<210> 84

<211> 21
<212> DNA
<213> Artificial sequence
<220>
<223> Oligonucleotide used as primer for PCR detection of MRP3 mRNA

<300>
<308> Unigene Hs.90786

<400> 84
cgtccagcac attgtttggg t

<200>
<210> 85
<211> 21
<212> DNA
<213> Artificial sequence
<220>
<223> Oligonucleotide used as primer for PCR detection of MRP4 mRNA

<300>
<308> Unigene Hs.139336

<400> 85
gatcgcagtg actgccctac t

<200>
<210> 86
<211> 22
<212> DNA
<213> Artificial sequence
<220>
<223> Oligonucleotide used as primer for PCR detection of MRP4 mRNA

<300>
<308> Unigene Hs.139336

<400> 86
gtggtgaagg tcacaaacac ga

<200>
<210> 87
<211> 21
<212> DNA
<213> Artificial sequence
<220>
<223> Oligonucleotide used as primer for PCR detection of MRP5 mRNA

<300>
<308> Unigene Hs.108660

<400> 87
acggaaagag gcacccatga g

<200>
<210> 88
<211> 21
<212> DNA
<213> Artificial sequence
<220>
<223> Oligonucleotide used as primer for PCR detection of MRP5 mRNA

<300>
<308> Unigene Hs.108660

<400> 88
tgttcccgct tccttgcttg a

<200>
<210> 89
<211> 20
<212> DNA

<213> Artificial sequence
<220>
<223> Oligonucleotide used as primer for PCR detection of MRP6 mRNA

<300>
<308> Unigene Hs.274260

<400> 89
tggatcgtgg tctgcttcgt

<200>
<210> 90
<211> 19
<212> DNA
<213> Artificial sequence
<220>
<223> Oligonucleotide used as primer for PCR detection of MRP6 mRNA

<300>
<308> Unigene Hs.274260

<400> 90
ttctcggcca ccagagtgt

<200>
<210> 91
<211> 22
<212> DNA
<213> Artificial sequence
<220>
<223> Oligonucleotide used as primer for PCR detection of BCRP mRNA

<300>
<308> Unigene Hs.194720

<400> 91

gccgtggaac tctttgtggt ag

<200>
<210> 92
<211> 22
<212> DNA
<213> Artificial sequence
<220>
<223> Oligonucleotide used as primer for PCR detection of BCRP mRNA

<300>
<308> Unigene Hs.194720

<400> 92
acagccaaga tgcaatggtt gt

<200>
<210> 93
<211> 23
<212> DNA
<213> Artificial sequence
<220>
<223> Oligonucleotide used as primer for PCR detection of COX-2
mRNA

<300>
<308> Unigene Hs.196384

<400> 93
agtccctgag catctacggt ttg

<200>
<210> 94
<211> 23
<212> DNA
<213> Artificial sequence

<220>
<223> Oligonucleotide used as primer for PCR detection of COX-2
mRNA

<300>
<308> Unigene Hs.196384

<400> 94
gaaggggatg ccagtgatag agg

<200>
<210> 95
<211> 21
<212> DNA
<213> Artificial sequence
<220>
<223> Oligonucleotide used as primer for PCR detection of iNOS mRNA

<300>
<308> Unigene Hs.46752

<400> 95
ccccacctca ggaaaacagt c

<200>
<210> 96
<211> 23
<212> DNA
<213> Artificial sequence
<220>
<223> Oligonucleotide used as primer for PCR detection of iNOS mRNA

<300>
<308> Unigene Hs.46752

<400> 96

ctctgtgtcc ttgagctggt aag

<200>
<210> 97
<211> 23
<212> DNA
<213> Artificial sequence
<220>
<223> Oligonucleotide used as primer for PCR detection of TGF-beta
mRNA

<300>
<308> Unigene Hs.1103

<400> 97
gcaacaattc ctggcgatac ctc

<200>
<210> 98
<211> 23
<212> DNA
<213> Artificial sequence
<220>
<223> Oligonucleotide used as primer for PCR detection of TGF-beta
mRNA

<300>
<308> Unigene Hs.1103

<400> 98
agttcttctc cgtggagctg aag

<200>
<210> 99
<211> 23
<212> DNA

<213> Artificial sequence
<220>
<223> Oligonucleotide used as primer for PCR detection of
beta-actin mRNA

<300>
<308> Unigene Hs.288061

<400> 99
gtaccactgg catcgtgatg gac

<200>
<210> 100
<211> 24
<212> DNA
<213> Artificial sequence
<220>
<223> Oligonucleotide used as primer for PCR detection of
beta-actin mRNA

<300>
<308> Unigene Hs.288061

<400> 100
gctcattgcc aatggtgatg acct